# NEW EUROPEAN PATENT SPECIFICATION

(11) **EP 2 525 811 B2**
(45) Date of publication and mention of the opposition decision: **27.02.2019**
(45) Mention of the grant of the patent: 16.03.2016
(21) Application number: 11701334.2
(22) Date of filing: 18.01.2011
(51) Int. Cl.: A61K 39/00, C07H 3/06, A61P 11/06, A61P 11/00, A61P 37/08, A61P 17/00, A61K 35/74, A61K 31/702

(54) **A COMPOSITION COMPRISING LACTOBACILLUS RHAMNOSUS HN001 AND PREBIOTICS FOR USE IN THE TREATMENT OF ALLERGIC LUNG DISEASE**
ZUSAMMENSETZUNG ENTHALTEND LACTOBACILLUS RHAMNOSUS HN001 UND PRÄBIOTIKA ZUR BEHANDLUNG VON ALLERGISCHER LUNGENKRANKHEIT
UNE COMPOSITION COMPRENANT DU LACTOBACILLUS RHAMNOSUS HN001 ET DES PRÉBIOTIQUES POUR LE TRAITEMENT DES MALADIES PULMONAIRES ALLERGIQUES

(30) Priority: 19.01.2010 US 296217 P
(43) Date of publication of application: 28.11.2012
(73) Proprietor: ABBOTT LABORATORIES, Abbott Park, IL 60064-3500 (US)
(72) Inventor: THOMAS, Debra, L, Columbus Ohio 43221 (US); BUCK, Rachael, H, Gahanna Ohio 43230 (US)
(74) Representative: Boult Wade Tennant LLP
(86) International application number: PCT/US2011/021506
(87) International publication number: WO 2011/090926

(56) References cited:
- WO-A1-2005/060937
- WO-A1-2007/123488
- WO-A1-2010/064930
- US-A1- 2008 299 255
- GOPAL PRAMOD K ET AL: "Utilisation of galacto-oligosaccharides as selective substrates for growth by lactic acid bacteria including Bifidobacterium lactis DR10 and Lactobacillus rhamnosus DR20", INTERNATIONAL DAIRY JOURNAL, vol. 11, no. 1-2, 2001, pages 19-25, XP002626803, ISSN: 0958-6946
- SHEIH YING-H ET AL: "Systemic immunity-enhancing effects in healthy subjects following dietary consumption of the lactic acid bacterium Lactobacillus rhamnosus HN001", JOURNAL OF THE AMERICAN COLLEGE OF NUTRITION, vol. 20, no. 2 with Supplement, April 2001 (2001-04), pages 149-156, XP002626804, ISSN: 0731-5724
- PROBIOTIC STUDY GROUP ET AL: "A differential effect of 2 probiotics in the prevention of eczema and atopy: A double-blind, randomized, placebo-controlled trial", JOURNAL OF ALLERGY AND CLINICAL IMMUNOLOGY, MOSBY, INC, US, vol. 122, no. 4, 1 October 2008 (2008-10-01), pages 788-794, XP025493925, ISSN: 0091-6749, DOI: DOI:10.1016/J.JACI.2008.07.011 [retrieved on 2008-08-31]
- KUKKONEN ET AL: "Probiotics and prebiotic galacto-oligosaccharides in the prevention of allergic diseases: A randomized, double-blind, placebo-controlled trial", JOURNAL OF ALLERGY AND CLINICAL IMMUNOLOGY, MOSBY, INC, US, vol. 119, no. 1, 1 January 2007 (2007-01-01), pages 192-198, XP022128226, ISSN: 0091-6749, DOI: DOI:10.1016/J.JACI.2006.09.009
- DEKKER J W ET AL: "Safety aspects of probiotic bacterial strains Lactobacillus rhamnosus HN001 and Bifidobacterium animalis subsp. lactis HN019 in human infants aged 0-2 years", INTERNATIONAL DAIRY JOURNAL, ELSEVIER APPLIED SCIENCE, BARKING, GB, vol. 19, no. 3, 1 March 2009 (2009-03-01), pages 149-154, XP025799021, ISSN: 0958-6946, DOI: DOI:10.1016/J.IDAIRYJ.2008.10.004 [retrieved on 2008-10-21]
- FELESZKO W ET AL: "Probiotic-induced suppression of allergic sensitization and airway inflammation is associated with an increase of T regulatory-dependent mechanisms in a murine model of asthma", CLINICAL AND EXPERIMENTAL ALLERGY, vol. 37, no. 4, April 2007 (2007-04), pages 498-505, XP002626805, ISSN: 0954-7894
- BOYLE R J ET AL: "Probiotics for the treatment of eczema: A systematic review", CLINICAL AND EXPERIMENTAL ALLERGY 2009 BLACKWELL PUBLISHING LTD GBR LNKD- DOI:10.1111/J.1365-2222.2009.03305.X, vol. 39, no. 8, August 2009 (2009-08), pages 1117-1127, XP002626806, ISSN: 0954-7894
- JOHANNSEN H ET AL: "Practical prebiotics, probiotics and synbiotics for allergists: How useful are they?", CLINICAL AND EXPERIMENTAL ALLERGY 2009 BLACKWELL PUBLISHING LTD GBR LNKD- DOI:10.1111/J.1365-2222.2009.03368.X, vol. 39, no. 12, December 2009 (2009-12), pages 1801-1814, XP002626807, ISSN: 0954-7894
- MORO G ET AL: "A mixture of prebiotic oligosaccharides reduces the incidence of atopic dermatitis during the first six months of age", ARCHIVES OF DISEASE IN CHILDHOOD, BMJ GROUP, GB, vol. 91, 27 July 2006 (2006-07-27), pages 814-819, XP002468866, ISSN: 1468-2044, DOI: 10.1136/ADC.2006.098251
- <<<N O N - C I T E D D O C U M E N T>>> SHEIH YING-H et al.: "Systemic immunity-enhancing effects in healthy subjects following dietary consumption of the lactic acid bacterium Lactobacillus rhamnosus HN 001", Journal of the American College of Nutrition, vol. 20, no. 2, April 2001 (2001-04), pages 149-156, XP002626804,
- <<<N O N - C I T E D D O C U M E N T>>> DEKKER JW et al.: "Safety aspects of probiotic bacterial strains Lactobacillus rhamnosus HN 001 and Bifidobacterium animalis subsp. lactis HN 019 in human infants aged 0-2 years", International Dairy Journal, vol. 19, no. 3, 1 March 2009 (2009-03-01), pages 149-154, XP025799021, Barking, GB
- <<<N O N - C I T E D D O C U M E N T>>> WICKENS K et al.: "A differential effect of 2 probiotics in the prevention of eczema and atopy: A double-blind, randomized, placebo-controlled trial", Journal of Allergy and Clinical Immunology, vol. 122, no. 4, 1 October 2008 (2008-10-01), pages 788-794, XP025493925, US
- <<<N O N - C I T E D D O C U M E N T>>> FANARO S et al.: "Galacto-oligosaccharides and long-chain fructo- oligosaccharides as prebiotics in infant formulas: A review", Acta Paediatrica, vol. 94, no. Suppl 449, 2005, pages 22-26, XP009109493,
- <<<N O N - C I T E D D O C U M E N T>>> FELESZKO W et al.: "Probiotic-induced suppression of allergic sensitization and airway inflammation is associated with an increase of T regulatory-dependent mechanisms in a murine model of asthma", Clinical and Experimental Allergy, vol. 37, no. 4, April 2007 (2007-04), pages 498-505, XP002626805,

## Description

### TECHNICAL FIELD

The present disclosure relates to a synbiotic composition comprising the probiotic *Lactobacillus rhamnosus* HN001 and a carbohydrate-based prebiotic such as fructooligosaccharides, galactooligosaccharides, human milk oligosaccharides, or combinations thereof, and the use of the composition for the prevention and/or treatment of allergic disease.

### BACKGROUND OF THE DISCLOSURE

Allergic diseases such as atopic dermatitis, allergic rhinitis, and asthma represent a significant segment of chronic disease in the world. For instance, it is estimated that 150 million people around the world suffer from asthma, while mortality has reached over 180,000 annually. In the United States alone there were an estimated 20.3 million asthmatics in 2001, and the number of asthmatics has leapt by over 60% since the early 1980s and deaths have doubled to 5,000 a year. *See* Li, et al., "Allergic Asthma: Symptoms and Treatment," World Allergy Organization Web site, accessed August 11, 2009.

The incidence of allergic diseases may be especially problematic in infants and during early childhood. Infants are devoid of intestinal flora at birth. As a result of contact with the mother during birth and subsequent feeding with human or formula milk, the intestinal flora rapidly develops. Generally, the intestinal flora of human milk-fed infants is primarily composed of Bifidobacteria and Lactobacilli. Bifidobacteria and Lactobacilli are considered to be important in maintaining a well-balanced intestinal microbiota, and it has been postulated that Bifidobacteria and Lactobacilli have several health-promoting effects, possibly playing a role in early immune system stimulation in the host.

Probiotics, including various strains of *Lactobacillus*, have been test administered for their efficacy in the treatment and prevention of allergy in infants. The role of *Lactobacillus* probiotics in allergy prevention is, however, controversial. For instance, some studies have found that administration of *Lactobacillus* probiotics do not reduce the incidence of atopic dermatitis when administered during pregnancy and early infancy, and in fact are associated with increased rates of recurrent episodes of wheezing bronchitis *(see* Kopp, et al., Pediatrics, 2008, Vol. 121, p. e850-e856, in which a *Lactobacillus rhamnosus GC* probiotic was administered). See also Taylor, et al., J. Allergy Clin. Immunol., 2007, Vol. 119, No. 1, p. 184-191, describing a study which found that early probiotic supplementation with *Lactobacillus acidophilous* did not reduce the risk of atopic dermatitis, and was associated with a significantly increased likelihood of developing associated wheezing in children in the second 6 months of life; and Kalliomaki, et al., J. Allergy Clin. Immunol., 2007, Vol. 119, No. 4, p. 1019-1021, describing a study which found that allergic rhinitis and asthma tended to be more common during the first 7 years of life for children whose mothers received *Lactobacillus rhamnosus GC* from four weeks prior to expected delivery and postnatally for 6 months.

It has now been discovered that a *Lactobacillus rhamnosus* HN001 probiotic, when administered to an individual in combination with certain carbohydrate-based prebiotics, can be used to treat and/or prevent not only allergic skin diseases, but unexpectedly and in contrast to previously reported studies with other species of *Lactobacillus,* also can be used to treat and/or prevent allergic lung diseases. This effect may also be achieved without prenatal administration of the *Lactobacillus rhamnosus* HN001 probiotic and the prebiotic.

### SUMMARY OF THE DISCLOSURE

The present disclosure is directed to compositions for treating or preventing allergic disease. In one embodiment, the composition comprises a probiotic comprising *Lactobacillus rhamnosus* HN001 and a prebiotic selected from the group consisting of fructooligosaccharides, galactooligosaccharides, human milk oligosaccharides, and combinations thereof. In one exemplary embodiment, the composition may be a nutritional formula or infant formula.

The present disclosure is further directed to methods of treating or preventing allergic disease in an individual in need of such treatment or prevention. In one method of the present disclosure, the individual is enterally administered an effective amount of a composition comprising a probiotic comprising *Lactobacillus rhamnosus* HN001, and a prebiotic selected from the group consisting of fructooligosaccharides, galactooligosaccharides, human milk oligosaccharides, and combinations thereof.

It has been unexpectedly found that *Lactobacillus rhamnosus* HN001 and a carbohydrate-based prebiotic selected from the group consisting of fructooligosaccharides, galactooligosaccharides, human milk oligosaccharides, and combinations thereof can be administered to an individual prior or subsequent to onset of an allergic disease to prevent the occurrence of the allergic disease and/or to reduce the severity of an allergic response.

### BRIEF DESCRIPTION OF THE DRAWINGS

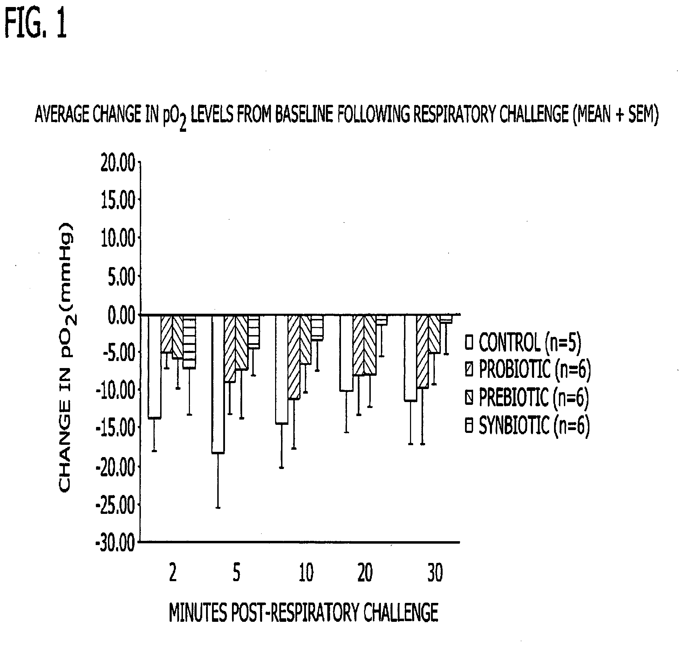

Figure 1 is a chart showing the average change in pO₂ levels from baseline at various times post-respiratory challenge in pigs orally administered a control chow, a chow comprising a *L. rhamnosus* HN001 probiotic, a chow comprising a fructooligosaccharide-based prebiotic, or a chow comprising both a *L. rhamnosus* HN001 probiotic and a fructooligosaccharide-based prebiotic (synbiotic composition), as discussed in the experiments discussed in the Examples.

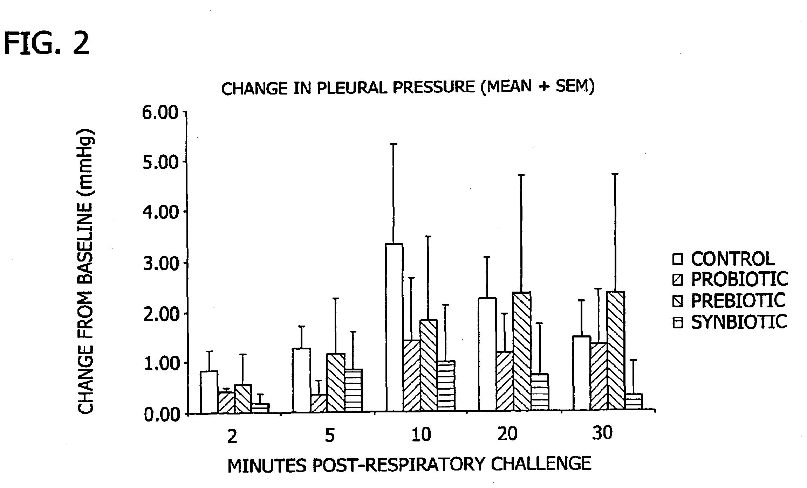

Figure 2 is a chart showing the average change in pleural pressure from baseline at various times post-respiratory challenge in pigs orally administered a control chow, a chow comprising a *L. rhamnosus* HN001 probiotic, a chow comprising a fructooligosaccharide-based prebiotic, or a chow comprising both a *L. rhamnosus* HN001 probiotic and a fructooligosaccharide-based prebiotic (synbiotic composition), as discussed in the Examples.

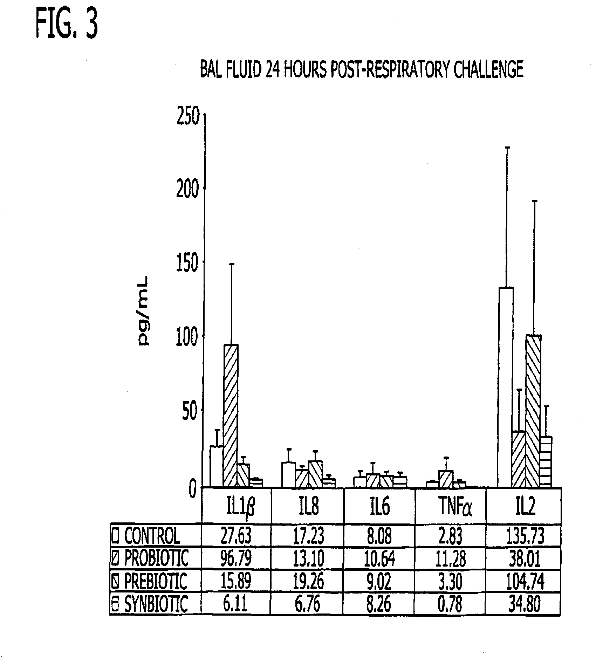

Figure 3 is a chart showing the average level of various cytokines in bronchoalveolar lavage fluid taken at 24 hours post-respiratory challenge from pigs orally administered a control chow, a chow comprising a *L. rhamnosus* HN001 probiotic, a chow comprising a fructooligosaccharide-based prebiotic, or a chow comprising both a *L. rhamnosus* HN001 probiotic and a fructooligosaccharide-based prebiotic (synbiotic composition), as discussed in the Examples.

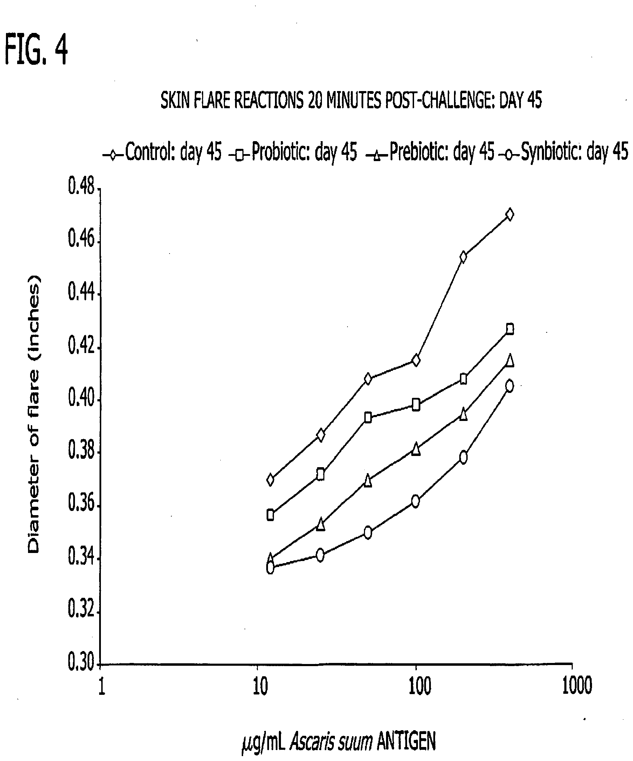

Figure 4 is a chart showing the average allergic skin flare reactions 20 minutes after challenge with various doses of antigen on day 45 for pigs administered a control chow (◇) as compared to pigs administered a chow comprising a *L. rhamnosus* HN001 probiotic (■), a chow comprising a galactooligosaccharide-based prebiotic (▲), or a chow comprising both a *L.* rhamnosus HN001 probiotic and a galactooligosaccharide-based prebiotic (●) (symbiotic composition) as discussed in the Examples.

### DETAILED DESCRIPTION OF THE DISCLOSURE

The present disclosure is directed to synbiotic compositions comprising the probiotic *Lactobacillus rhamnosus* HN001 and a carbohydrate-based prebiotic selected from the group consisting of fructooligosaccharides, galactooligosaccharides, human milk oligosaccharides, and combinations thereof. The synbiotic compositions may be formulated as nutritional formulas or infant formulas, bars, or as a supplement for administration to infants, children, or adults. The present disclosure further provides methods of treating and/or preventing allergic disease in an individual in need of such treatment or prevention, by administering to the individual a synbiotic composition of the present disclosure. These and other essential or optional elements or limitations of the symbiotic compositions and methods of the present disclosure are described in detail hereafter.

The term "infant" as used herein, unless otherwise specified, refers to children not more than about one year of age, and includes infants from 0 to about 4 months of age, infants from about 4 to about 8 months of age, infants from about 8 to about 12 months of age, low birth weight infants at less than 2,500 grams at birth, and preterm infants born at less than about 37 weeks gestational age, typically from about 26 weeks to about 34 weeks gestational age. The term "child" or "children" as used herein refers to children not more than 12 years of age, and includes children from about 12 months to about 12 years of age. The term "adult" as used herein refers to adults and children about 12 years and older.

The term "infant formula" as used herein, unless otherwise specified, refers to a nutritional composition designed for infants that contains sufficient nutrients such as proteins, carbohydrates, lipids, vitamins, and minerals to potentially serve as a supplemental, primary, or sole source of nutrition.

The term "nutritional formula" as used herein, unless otherwise specified, refers to a nutritional composition designed for infants, toddlers, children, adults, or combinations thereof, that contains sufficient nutrients such as proteins, carbohydrates, lipids, vitamins, minerals, and electrolytes to potentially serve as a supplemental, primary, or sole source of nutrition.

The term "ready-to-feed" as used herein, unless otherwise specified, refers to nutritional formulas in liquid form suitable for administration, including reconstituted powders, diluted concentrates, and manufactured liquids.

The term "prebiotic" as used herein, unless otherwise specified, refers to a substantially non-digestible food ingredient that selectively stimulates the growth and/or activity of one or more bacterial species in the digestive system which are beneficial to the health of the host.

The term "probiotic" as used herein, unless otherwise specified, refers to a live or dead microorganism which when administered in adequate amounts confers a health benefit on the host. An example of a probiotic used herein is *Lactobacillus rhamnosus* HN001.

The term "indigestible oligosaccharides" as used herein, unless otherwise specified, refers to saccharides which are not or only partially digested in the intestine by the action of acids or digestive enzymes present in the human upper digestive tract (small intestine and stomach) but which are fermented by the human intestinal flora.

All percentages, parts and ratios as used herein, are by weight of the total composition, unless otherwise specified. All such weights as they pertain to listed ingredients are based on the active level and, therefore, do not include solvents or by-products that may be included in commercially available materials, unless otherwise specified.

Numerical ranges as used herein are intended to include every number and subset of numbers contained within that range, whether specifically disclosed or not. Further, these numerical ranges should be construed as providing support for a claim directed to any number or subset of numbers in that range. For example, a disclosure of from 1 to 10 should be construed as supporting a range of from 2 to 8, from 3 to 7, from 5 to 6, from 1 to 9, from 3.6 to 4.6, from 3.5 to 9.9, and so forth.

All references to singular characteristics or limitations of the present disclosure shall include the corresponding plural characteristic or limitation, and vice versa, unless otherwise specified or clearly implied to the contrary by the context in which the reference is made.

All combinations of method or process steps as used herein can be performed in any order, unless otherwise specified or clearly implied to the contrary by the context in which the referenced combination is made.

The compositions of the present disclosure, including nutritional and infant formulas, may also be substantially free of any optional or selected essential ingredient or feature described herein, provided that the remaining composition still contains all of the required ingredients or features as described herein. In this context, and unless otherwise specified, the term "substantially free" means that the selected composition contains less than a functional amount of the optional ingredient, typically less than 0.1% by weight, and also including zero percent by weight of such optional or selected essential ingredient.

The compositions, nutritional formulas, infant formulas, and corresponding methods of the present disclosure can comprise, consist of, or consist essentially of the essential elements and limitations of the disclosure as described herein, as well as any additional or optional ingredients, components, or limitations described herein or otherwise useful in nutritional formula applications.

### PROBIOTIC

The synbiotic compositions of the present disclosure include a probiotic, specifically the probiotic *Lactobacillus rhamnosus* strain HN001 (*L. rhamnosus* HN001), also referred to as *Lactobacillus rhamnosus* DR20. It has surprisingly been discovered that the probiotic *L. rhamnosus* HN001 is effective at treating and/or preventing allergic diseases in an individual when enterally administered to the individual in combination with certain carbohydrate-based prebiotics as described herein.

*L.rhamnosus* HN001 are heterofermentative bacteria that are Gram positive, non-motile, non-spore forming, catalase negative, facultative anaerobic rods exhibiting an optimal growth temperature of 371°C and an optimum pH of 6.0-6. 5. *See* WO 2004/031389, *L. rhamnosus* HN001 are described in more detail in U.S. Patent No. 6,379,663, *L. rhamnosus* HN001 has previously been deposited with the Australian Government Analytical Laboratories (AGAL), deposit number NM97/09514, on August 18, 1997 by the New Zealand Dairy Board, now known as Fonterra (Auckland, New Zealand).

The *L. rhamnosus* HN001 probiotic may be provided as viable or non-viable cells, although it is generally preferably provided as viable cells; i.e., live cells which reach the intestine of an individual ingesting the probiotic. The probiotic may be in various forms including, for example, freeze-dried cells, wet cells, a culture fluid, yogurts, or combinations thereof. In one embodiment, freeze-dried cells are preferred.

The synbiotic compositions of the present disclosure may be in powder, liquid or bar form and may be included into a nutritional formula, as described hereinafter. The compositions may comprise any amount of *Lactobacillus rhamnosus* HN001 probiotic effective for treating and/or preventing allergic disease when enterally administered to an individual in combination with the prebiotic of the present disclosure. Typically, the compositions will comprise probiotic in sufficient amounts to provide a daily dose of from about 10⁶ colony forming units (cfu) to about 10¹² cfu, or from about 10⁶ cfu to about 10¹⁰ cfu, or from about 10⁸ cfu to about 10¹² cfu, or from about 10⁸ cfu to about 10¹⁰ cfu of probiotic to an individual upon ingestion of the composition. In some embodiments, the compositions will comprise probiotic sufficient to provide a daily dose of about 10⁶ cfu, or about 10⁷ cfu, or about 10⁸ cfu, or about 10⁹ cfu, or about 10¹⁰ cfu, or about 10¹¹ cfu or about 10¹² cfu to an individual upon ingestion of the composition.

When the synbiotic composition is formulated as a nutritional formula, the nutritional formula may comprise from about 10⁴ cfu to about 10¹⁰ cfu of probiotic per gram dry weight of the nutritional formula, or from about 10⁴ cfu to about 10⁹ cfu per gram dry weight of the nutritional formula, or from about 10⁴ cfu to about 10⁸ cfu of probiotic per gram dry weight of the nutritional formula, or from about 10⁴ cfu to about 10⁷ cfu of probiotic per gram dry weight of the nutritional formula, or from about 10⁴ cfu to about 10⁶ cfu of probiotic per gram dry weight of the nutritional formula, or from about 10⁴ cfu to about 10⁵ cfu of probiotic per gram dry weight of the nutritional formula. In another embodiment, the nutritional formula may comprise from about 10⁶ cfu to about 10⁸ cfu of probiotic per gram dry weight of the nutritional formula, or from about 10⁶ cfu to about 10⁷ cfu of probiotic per gram dry weight of the nutritional formula. In another embodiment, the nutritional formula may comprise about 10⁴ cfu of probiotic per gram dry weight of the nutritional formula, or about 10⁵ cfu of probiotic per gram dry weight of the nutritional formula, or about 10⁶ cfu of probiotic per gram dry weight of the nutritional formula, or about 10⁷ cfu of probiotic per gram dry weight of the nutritional formula, or about 10⁸ cfu of probiotic per gram dry weight of the nutritional formula, or about 10⁹ cfu of probiotic per gram dry weight of the nutritional formula, or about 10¹⁰ cfu of probiotic per gram dry weight of the nutritional formula.

### PREBIOTIC

The synbiotic compositions of the present disclosure include at least one carbohydrate-based prebiotic. The prebiotics of the present disclosure are indigestible oligosaccharides that selectively stimulate the growth and/or activity of bacteria in the digestive system, and in particular *L. rhamnosus* HN001. It has surprisingly been discovered that the prebiotics of the present disclosure act in combination with the *L. rhamnosus* HN001 probiotic to reduce the incidence or severity of allergic diseases and prevent the onset of allergic diseases.

The carbohydrate based prebiotic of the present disclosure is selected from the group consisting of long chain fructooligosaccharides, short chain fructooligosaccharides (generally referred to as fructooligosaccharides), galactose-containing oligosaccharides (generally referred to as galactooligosaccharides), human milk oligosaccharides, and combinations thereof.

Fructooligosaccharides (FOS), which are also referred to herein as fructooligosaccharide-based prebiotics, are saccharides comprising β-linked fructose units, which are preferably linked by β(2,1) and/or β(2,6) glycosidic linkages. Examples of suitable fructooligosaccharides include inulin, levan, and graminan. In general, inulin, levan, and graminan differ in the amount of branching that is present in their fructose chains and in the types of bonds connecting the individual fructose units. For example, levans generally consist of chains of fructose units that are typically connected by a β(2-6) bond. Although levans may occur as linear chain carbohydrates, they are more typically composed of branched fructose chains. In contrast, inulins generally consist of linear chains of fructose units that are typically connected by β(2-1) linkages. Graminans, or mixed type fructans, may comprise both β(2-1) and β(2-6) linkage bonds between fructose units. The fructooligosaccharide of the present disclosure preferably contains a β(2,1) glycosidic linked glucose at the reducing end.

Fructooligosaccharides may be found widely distributed in nature. For example, inulin may be found as a plant storage carbohydrate, and is common to plants of the Composite family. Inulin may be derived from a variety of plants, such as Jerusalem artichoke and Dahlia tuber, and is a major constituent of some herbs, such as burdock root, dandelion root, elecampane root, chicory root, and codonopsis, among others. Fructooligosaccharides are also commercially available as, for example, Raftilose® (Orafti), Actilight (Beghin-Meiji), or Synergy 1® (Orafti) among others.

The fructooligosaccharide of the present disclosure will typically have a degree of polymerization of from 2 to about 20. Preferably the fructooligosaccharide has a degree of polymerization of from 2 to about 10, or even from 2 to 7. It should be understood that not all fructooligosaccharides present in a composition of the present disclosure need to have the same degree of polymerization. For instance, the term "fructooligosaccharide" or "FOS" may also refer to a mixture of fructooligosaccharides having varying chain lengths; that is, long chain lengths and/or short chain lengths.

Another example of a suitable prebiotic is a galactose-containing oligosaccharide, commonly referred to as a galactooligosaccharide (GOS). GOS are indigestible oligosaccharides containing one or more galactose molecule and one molecule of glucose connected through β(1,4) and/or β(1,6) glycosidic bonds. The GOS used in the compositions of the present disclosure may be selected from β-galactooligosaccharides, α-galactooligosaccharides, and (arabino-) galactans. In some embodiment, the GOS may be a trans-galactooligosaccharide (TOS). The GOS may be represented by the formula: [galactose]ₙ-glucose, wherein n is an integer between 1 and 20, and preferably is selected from 2, 3, 4, 5, 6, 7, 8, 9, or 10. The term "galactooligosaccharide" or "GOS" may also refer to a mixture of galactooligosaccharides having different chain lengths; that is, long chain lengths and/or short chain lengths. Galactooligosaccharides are also commercially available as, for example, Vivinal® GOS (Friesland) and GOS (Clasado).

Another example of a suitable prebiotic is human milk oligosaccharide. The term "human milk oligosaccharide" or "HMO" refers generally to a number of complex carbohydrates found in human milk. Monomers found in milk oligosaccharides typically include D-glucose (Glc), D-galactose (Gal), N-acetylglucosamine (GlcNAC), L-fucose (Fuc), and sialic acid [N-acetylneuraminic acid (NeuAc)]. Elongation of these monomers may be achieved by attachment of GlcNAc residues linked in β1-3 or β1-4 linkage to a Gal residue followed by further addition of Gal in a β-1-3 or β-1-4 bond. Most HMOs carry lactose at their reducing end. From these monomers, a large number of core structures may be formed. Further variations may occur due to the attachment of lactosamine, Fuc, and/or NeuAc.

Examples of suitable HMOs that may be included in the compositions of the instant disclosure include lacto-N-tetraose, lacto-N-neotetraose, lacto-N-fucopentaose I, lacto-N-fucopentaose II, lacto-N-fucopentaose III, lacto-N-fucopentaose V, lacto-N-hexaose, para-lacto-N-hexaose, lacto-N-neohexaose, para-lacto-N-neohexaose, monofucosyllacto-N-hexaose II, isomeric fucosylated lacto-N-hexaose (1), monofucosyllacto-N-hexaose, isomeric fucosylated lacto-N-hexaose (3), isomeric fucosylated lacto-N-hexaose (2), difucosyl-para-lacto-N-neohexaose, difucosyl-para-lacto-N-hexaose, difucosyllacto-N-hexaose, lacto-N-neoocataose, para-lacto-N-octanose, iso-lacto-N-octaose, lacto-N-octaose, monofucosyllactoneoocataose, monofucosyllacto-N-ocataose, difucosyllacto-N-octaose I, difucosyllacto-N-octaose II, difucosyllacto-N-neoocataose II, difucosyllacto-N-neoocataose I, lacto-N-decaose, trifucosyllacto-N-neooctaose, trifucosyllacto-N-octaose, trifucosyl-iso-lacto-N-octaose, and combinations thereof. These HMOs are described more fully in U.S. Patent Application No. 2009/0098240. Other suitable examples of HMOs that may be included in the compositions of the present disclosure include lacto-N-difuco-hexaose II, sialylα(2-3) lactose, sialylα(2-6) lactose, sialyl-lacto-N-tetraose a, sialyllacto-N-tetraose b, sialyl-lacto-N-tetraose c, sialyl-fucosyl-lacto-N-tetraose I, sialylfucosyl-lacto-N-tetraose II, and disialyl-lacto-N-tetraose.

The HMO may have a degree of polymerization of from 2 to about 20. Preferably the HMO has a degree of polymerization of from 2 to about 10. The term "human milk oligosaccharide" or "HMO" may also refer to a mixture of human milk oligosaccharides having different chain lengths.

HMOs can be derived using any number of sources and methods known to those of skill in the art. For example, HMOs can be purified from human milk using methods known in the art. One such method for extraction of oligosaccharides from pooled mother's milk entails the centrifugation of milk at 5,000xg for 30 minutes at 4°C and fat removal. Ethanol may then be added to precipitate proteins. After centrifugation to sediment precipitated protein, the resulting solvent is collected and dried by rotary evaporation. The resulting material may be adjusted to the appropriate pH of 6.8 with phosphate buffer and β-galactosidase is added. After incubation, the solution is extracted with chloroform-methanol, and the aqueous layer is collected. Monosaccharides and disaccharides are removed by selective adsorption of HMOs using solid phase extraction with graphitized nonporous carbon cartridges. The retained oligosaccharides can be eluted with water-acetonitrile (60:40) with 0.01% trifluoroacetic acid. Individual HMOs can be further separated using methods known in the art such as capillary electrophoresis, HPLC (e.g., high-performance anion-exchange chromatography with pulsed amperometric detection; HPAEC-PAD), and thin layer chromatography. See, e.g., U.S. Patent Application No. 2009/0098240.

Alternately, enzymatic methods can be used to synthesize HMOs. In general, any oligosaccharide biosynthetic enzyme or catabolic enzyme (with the reaction running in reverse) that converts a substrate into any of the HMO structures (or their intermediates) may be used to prepare the HMOs. Another approach to the synthesis of the HMOs entails the chemical or enzymatic synthesis of or isolation of oligosaccharide backbones containing Lacto-N-biose, or Lacto-N-tretrose from non-human mammalian milk sources (e.g., cows, sheep, buffalo, goat, etc.) and enzymatically adding Lacto-N-biose, Fucose and Sialic Acid units as necessary to arrive at HMO structures. Examples of such oligosaccharide modifying enzymes include sialidases, silate O-Acetylesterases, N-Acetylneuraminate lyases, N-acetyl-beta-hexosaminidase, beta-galactosidases, N-acetylmannosamine-6-phosphate 2-epimerases, alpha-L-fucosidases, and fucose dissimilation pathway proteins, among others, which may be used to catalyze a biosynthetic reaction under the appropriate conditions. Alternatively, conventional chemical methods may be used for the de novo organic synthesis of or conversion of pre-existing oligosaccharides into HMO structures. These techniques for synthesizing HMOs are described in more detail in U.S. Patent Application No. 2009/0098240.

In one exemplary embodiment, the prebiotic used in combination with the *L. rhamnosus* HN001 is a combination of two or more of galactooligosaccharides, fructooligosaccharides, and/or human milk oligosaccharides.

The synbiotic compositions of the present disclosure may be in powder or liquid form and/or may be included into a nutritional or infant formula, as described hereinafter. The compositions may comprise any amount of prebiotic effective for treating and/or preventing allergic disease when enterally administered to an individual in combination with the *Lactobacillus rhamnosus* HN001 probiotic. Typically, the compositions will comprise prebiotic in sufficient amounts to provide from about 0.4 grams per day of prebiotic to about 9.2 grams per day of prebiotic, or from about 1.5 grams per day of prebiotic to about 8.5 grams per day of prebiotic, or about 1.5 grams per day of prebiotic to about 7.0 grams per day of prebiotic, or about 1.5 grams per day of prebiotic to about 6.0 grams per day of prebiotic, or about 1.5 grams per day of prebiotic to about 4.0 grams per day of prebiotic, or about 1.5 grams per day of prebiotic to about 2.0 grams per day of prebiotic to an individual upon ingestion of the composition. In another embodiment, the compositions will comprise prebiotic in sufficient amounts to provide from about 3.0 grams per day of prebiotic to about 6.0 grams per day of prebiotic, or from about 3.0 grams per day of prebiotic to about 5.0 grams per day of prebiotic, or from about 3.0 grams per day of prebiotic to about 4.0 grams per day of prebiotic to an individual upon ingestion of the composition. In another embodiment, the compositions will comprise prebiotic in sufficient amounts to provide about 1.5 grams per day of prebiotic, or 2.0 grams per day of prebiotic, or 2.5 grams per day of prebiotic, or 3.0 grams per day of prebiotic, or 3.5 grams per day of prebiotic, or 4.0 grams per day of prebiotic, or 4.5 grams per day of prebiotic, or 5.0 grams per day of prebiotic, or 5.5 grams per day of prebiotic, or 6.0 grams per day of prebiotic, or 6.5 grams per day of prebiotic, or 7.0 grams per day of prebiotic, or 7.5 grams per day of prebiotic, or 8.0 grams per day of prebiotic, or 8.5 grams per day of prebiotic, or 9.0 grams per day of prebiotic, or 9.2 grams per day of prebiotic to an individual upon ingestion of the composition.

When the synbiotic composition is formulated as a nutritional or infant formula, the nutritional or infant formula may comprise from about 0.35 grams prebiotic per 100 grams nutritional to about 9.2 grams prebiotic per 100 grams nutritional, or from about 1.5 grams prebiotic per 100 grams nutritional to about 7.0 grams prebiotic per 100 grams nutritional, or from about 1.5 grams prebiotic per 100 grams nutritional to about 6.0 grams prebiotic per 100 grams nutritional, and preferably from about 3.0 grams prebiotic per 100 grams nutritional to about 6.0 grams prebiotic to about 100 grams nutritional.

### NUTRIENTS

The synbiotic compositions of the present disclosure can be incorporated into any food or beverage that can be consumed by human infants or adults or animals. Thus, in one aspect, the composition of the present disclosure is formulated as a nutritional or infant formula. The nutritional or infant formulas of the present disclosure may comprise sufficient types and amounts of nutrients to meet the targeted dietary needs of the intended user. These nutritional or infant formulas may therefore comprise protein, carbohydrate, and a lipid component (all either organic or non-organic) in addition to the probiotic and prebiotic discussed above. The nutritional or infant formulas may also include vitamins, minerals, or other ingredients suitable for use in nutritional formulas.

For example, when the nutritional formula is an adult formula, the protein component may comprise from about 10% to about 80% of the total caloric content of said nutritional formula; the carbohydrate component may comprise from about 10% to about 70% of the caloric content of said nutritional formula; and the lipid component may comprise from about 5% to about 50% of the total caloric content of said nutritional formula. The nutritional formula may be in liquid or powder form. These ranges are provided as examples only, and are not intended to be limiting.

When the nutritional formula is a non-adult formula, such as an infant formula, the non-adult formula includes those embodiments in which the protein component may comprise from about 7.5% to about 25% of the caloric content of the nutritional formula; the carbohydrate component may comprise from about 35% to about 50% of the total caloric content of the nutritional formula; and the lipid component may comprise from about 30% to about 60% of the total caloric content of the nutritional formula. These ranges are provided as examples only, and are not intended to be limiting. Additional suitable ranges are noted in the following Table.

| **Nutrient*** | **1^{st} Embodiment** | **2^{nd} Embodiment** | **3^{rd} Embodiment** |
|---|---|---|---|
| Carbohydrates: % total calories | 20-85 | 30-60 | 35-55 |
| Lipid: % total calories | 5-70 | 20-60 | 25-50 |
| Protein: % total calories | 2-75 | 5-50 | 7-40 |
| *all numerical values preceded by the term "about" | | | |

Many different sources and types of carbohydrates, lipids, proteins, minerals and vitamins are known and can be used in the nutritional formulas of the present disclosure, provided that such nutrients are compatible with the added ingredients in the selected formula, are safe for their intended use, and do not otherwise unduly impair product performance.

Carbohydrates suitable for use in the nutritional formulas of the present disclosure can be simple, complex, or variations or combinations thereof. Non-limiting examples of suitable carbohydrates include hydrolyzed, intact, naturally and/or chemically modified cornstarch, maltodextrin, glucose polymers, sucrose, com syrup, corn syrup solids, rice or potato derived carbohydrate, glucose, fructose, lactose, high fructose corn syrup and indigestible oligosaccharides such as fructooligosaccharides (FOS), and combinations thereof.

Non-limiting examples of proteins suitable for use in the nutritional formulas include extensively hydrolyzed, partially hydrolyzed or non-hydrolyzed proteins or protein sources, and can be derived from any known or otherwise suitable source such as milk (e.g., casein, whey), animal (e.g., meat, fish), cereal (e.g., rice, corn), vegetable (e.g., soy), or combinations thereof. The proteins for use herein can also include, or be entirely or partially replaced by, free amino acids known for use in nutritional formulas, non-limiting examples of which include tryptophan, glutamine, tyrosine, methionine, cysteine, arginine, and combinations thereof. Other (non-protein) amino acids typically added to nutritional formulas include carnitine and taurine. In some cases, the D-forms of the amino acids are considered as nutritionally equivalent to the L-forms, and isomer mixtures are used to lower cost (for example, D,L-methionine).

Non-limiting examples of lipids suitable for use in the nutritional formulas include coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils, long-chain polyunsaturated fatty acids such as arachidonic acid (ARA), docosahexaenoic acid (DHA), and eicosapentaenoic acid (EPA), and combinations thereof.

In addition to these food grade oils, structured lipids may be incorporated into the nutritional formulas if desired. Structured lipids are known in the art, descriptions of which can be found in INFORM, Vol. 8, no. 10, page 1004, Structured lipids allow fat tailoring (October 1997); and U.S. Pat. No. 4,871,768. Structured lipids are predominantly triacylglycerols containing mixtures of medium and long chain fatty acids on the same glycerol nucleus. Structured lipids are also described in U.S. Pat. No. 6,160,007.

The nutritional formulas of the present disclosure may further comprise any of a variety of vitamins in addition to the components described above. Non-limiting examples of vitamins include vitamin A, vitamin D, vitamin E, vitamin K, thiamine, riboflavin, pyridoxine, vitamin B 12, niacin, folic acid, pantothenic acid, biotin, vitamin C, choline, chromium, camitine, inositol, salts and derivatives thereof, and combinations thereof.

The nutritional formulas may further comprise any of a variety of minerals, non-limiting examples of which include calcium, phosphorus, magnesium, iron, zinc, manganese, copper, iodine, sodium, potassium, chloride, and combinations thereof.

The infant formula embodiments of the present disclosure preferably comprise nutrients in accordance with the relevant infant formula guidelines for the targeted consumer or user population, as example of which would be the Infant Formula Act, 21 U.S.C. Section 350(a).

The nutritional formulas of the present disclosure include those embodiments containing the carbohydrate, lipid, and protein concentrations described in Table 1 (Nutritional Formula Macronutrients).

**Table 1***

| Nutrient | Embodiment | g/100 kcal | g/100 g total solids | g/L (as fed) |
|---|---|---|---|---|
| Carbohydrate | 1^{st} Embodiment | 8-16 | 30-90 | 54-108 |
| | 2^{nd} Embodiment | 9-13 | 45-60 | 57-79 |
| | 3^{nd} Embodiment | 15-19 | 63-81 | 157-203 |
| Lipid | 1^{st} Embodiment | 3-8 | 15-42 | 20-54 |
| | 2^{nd} Embodiment | 4-6.6 | 20-30 | 27-45 |
| | 3^{rd} Embodiment | 2-5 | 8-21 | 20-53 |
| Protein | 1^{st} Embodiment | 1-3.9 | 8-20.5 | 7-24 |
| | 2^{nd} Embodiment | 1.5-3.4 | 10-17 | 10-23 |
| | 3^{rd} Embodiment | 3.5-6.0 | 14.8-25.3 | 37-63 |
| *all numerical values preceded by the term "about" | | | | |

The nutritional formulas of the present disclosure include those embodiments that comprise per 100 kcal of reconstituted formula one or more of the following: vitamin A (from about 250 to about 1250 IU), vitamin D (from about 40 to about 150 IU), vitamin K (greater than about 4 mcg), vitamin E (at least about 0.3 IU), vitamin C (at least about 8 mg), thiamine (at least about 8 mcg), vitamin B 12 (at least about 0.15 mcg), niacin (at least about 250 mcg), folic acid (at least about 4 mcg), pantothenic acid (at least about 300 mcg), biotin (at least about 1.5 mcg), choline (at least about 7 mg), and inositol (at least about 4 mg).

The nutritional formulas of the present disclosure include those embodiments that comprise per 100 kcal of reconstituted formula one or more of the following: calcium (at least about 50 mg), phosphorus (at least about 25 mg), magnesium (at least about 6 mg), iron (at least about 0.15 mg), iodine (at least about 5 mcg), zinc (at least about 0.5 mg), copper (at least about 60 mcg), manganese (at least about 5 mcg), sodium (from about 20 to about 60 mg), potassium (from about 80 to about 200 mg), and chloride (from about 55 to about 150 mg).

### OPTIONAL INGREDIENTS

The nutritional formulas of the present disclosure may further comprise other optional components that may modify the physical, chemical, aesthetic or processing characteristics of the formulas or serve as pharmaceutical or additional nutritional components when used in the targeted population. Many such optional ingredients are known or other suitable for use in food and nutritional products, including infant formulas, and may also be used in the nutritional formulas of the present disclosure, provided that such optional materials are compatible with the essential materials described herein, are safe for their intended use, and do not otherwise unduly impair product performance.

Non-limiting examples of such optional ingredients include preservatives, anti-oxidants, emulsifying agents, buffers, colorants, flavors, nucleotides, and nucleosides, additional probiotics, additional prebiotics, lactoferrin, and related derivatives, thickening agents and stabilizers, and so forth.

### PRODUCT FORM

The nutritional formulas of the present disclosure may be prepared as any product form suitable for use in humans, including liquid or powdered complete nutritionals, liquid or powdered supplements (such as a supplement that can be mixed with a drink), reconstitutable powders, ready-to-feed liquids, bars, and dilutable liquid concentrates, which product forms are all well known in the nutritional formula arts.

The nutritional formulas of the present disclosure may have any caloric density suitable for the targeted or intended patient population, or provide such a density upon reconstitution of a powder embodiment or upon dilution of a liquid concentrate embodiment. Most common caloric densities for the infant formula embodiments of the present disclosure are generally at least about 19 kcal/fl oz (660 kcal/liter), more typically from about 20 kcal/fl oz (675-680 kcal/liter) to about 25 kcal/fl oz (820 kcal/liter), even more typically from about 20 kcal/fl oz (675-680 kcal/liter) to about 24 kcal/fl oz (800-810 kcal/liter). Generally, the 22-24 kcal/fl oz formulas are more commonly used in pre-term or low birth weight infants, and the 20-21 kcal/fl oz (675-680 to 700 kcal/liter) formulas are more often used in term infants. Non-infant and adult nutritional formulas may have any caloric density suitable for the targeted or intended population.

For nutritional powder embodiments of the present disclosure, such powders are typically in the form of flowable or substantially flowable particulate compositions, or at least particulate compositions that can be easily scooped and measured with a spoon or similar other device, wherein the compositions can easily be reconstituted by the intended user with a suitable aqueous fluid, typically water, to form a liquid nutritional formula for immediate oral or enteral use. In this context, "immediate" use generally means within about 48 hours, most typically within about 24 hours, preferably right after reconstitution. These powder embodiments include spray dried, agglomerated, dry mixed or other known or otherwise effective particulate form. The quantity of a nutritional powder required to produce a volume suitable for one serving may vary.

The nutritional formulas of the present disclosure may be packaged and sealed in single or multi-use containers, and then stored under ambient conditions for up to about 36 months or longer, more typically from about 12 to about 24 months. For multi-use containers, these packages can be opened and then covered for repeated use by the ultimate user, provided that the covered package is then stored under ambient conditions (e.g., avoid extreme temperatures) and the contents used within about one month or so.

### METHODS OF USE

The synbiotic compositions of the present disclosure may be used to treat and/or prevent allergic disease, particularly in individuals in need of such treatment or prevention, such as individuals afflicted with or otherwise at risk of developing allergic disease. These compositions can also be administered to any individual as a nutrition source, e.g., in the form of a nutritional formula, especially in those in whom protection against an allergic disease is desirable.

The compositions and methods of the present disclosure may be directed to any individual, including humans and other mammals such as dogs, cats, rodents, cows, sheep, swine, goats, horses and other hoofed animals, and so forth. Healthy individuals, including those at risk of developing allergic disease, may also be administered the compositions.

The compositions of the present disclosure may be used to prevent allergic disease by administering the compositions to an individual prior to onset of the allergic disease or symptoms thereof. For example, the compositions may be administered to an individual before, during, or after exposure to an allergen that may trigger an allergic disease or allergic response to protect against development of the allergic disease and/or to reduce the severity of the allergic response.

Thus in one aspect, the present disclosure is directed to a method of preventing allergic disease in an individual. The method comprises administering to the individual an effective amount of a synbiotic composition of the present disclosure. An "effective amount" of the synbiotic composition is any amount effective to achieve the desired treatment and/or prevention of an allergic disease, including a reduction (or elimination) of the severity of the symptoms of the allergic disease. As discussed herein, the composition may be administered prior to the onset of the allergic disease to protect against development of the allergic disease and/or to reduce the severity of an allergic response. Preferably, the composition is enterally administered.

The individual to whom the composition is administered may be, for example, one who is susceptible to development of the allergic disease. Thus in one aspect, the method may further comprise identifying an individual susceptible to development of an allergic disease. Individuals susceptible to development of allergic disease may include, for example, individuals who have a family history of one or more allergic diseases, individuals who have previously exhibited symptoms of an allergic disease, and/or individuals who are sensitive to allergens, including seasonal allergens. Such individuals may be identified by any means known in the art, including but not limited to examination of the medical and/or family history of an individual, and examination of an individual for signs of allergic responses to allergens.

The compositions of the present disclosure may also be used to treat allergic disease, by administering the compositions to an individual during or subsequent to onset of the allergic disease or symptoms thereof. For example, the compositions may be administered to an individual subsequent to onset of the allergic disease or symptoms thereof to reduce the severity of the allergic response and/or to reduce or eliminate symptoms of the allergic disease.

Thus in another aspect, the present disclosure is directed to a method of treating allergic disease or symptoms thereof in an individual. The method comprises administering to the individual an effective amount of a synbiotic composition of the present disclosure. As discussed herein, the composition may be administered subsequent to the onset of the allergic disease or symptoms thereof to reduce the severity of an allergic response and/or to reduce or eliminate symptoms of the allergic disease. Preferably, the composition is enterally administered. The individual to whom the composition is administered may be, for example, one who is susceptible to development of the allergic disease, as discussed above, and/or an individual exhibiting one or more symptoms of an allergic disease.

The compositions of the instant disclosure may be used to treat and/or prevent allergic diseases, including allergic lung diseases, allergic skin diseases, allergic rhinitis, and food allergies. Allergic diseases may result from exposure or reexposure to an allergen, such as pollens, dust mites, animal danders, mold spores, dusts, insects, food allergens, and the like.

Allergic lung diseases include asthma, such as atopic asthma, as well as hypersensitive pneumonitis, and other respiratory diseases that may be caused by allergens. Asthma typically involves intermittent reversible episodes of airflow obstruction characterized by cough, wheezing, chest tightness, and/or dyspnea. Symptoms of severe asthma include severe chest tightness, cough, sensation of air hunger, inability to lie flat, insomnia and severe fatigue. The signs of severe asthma also include use of accessory muscles of respiration, hyperinflation of the chest, tachypnea, tachycardia, sweating, diaphoresis, obtundation, apprehensive appearance, wheezing, inability to complete sentences and difficulty in lying down. Hayfever is typically a seasonal condition resulting from allergy to grasses and pollens, and is oftentimes characterized by sneezing, or in more severe cases inflammation and the development of polyps in the nasal passages. Hypersensitivity pneumonitis may be characterized by inflammation of the smaller bronchioles in the lungs, alveolar wall edema, and in some instances airflow obstruction due to smooth muscle contraction.

Allergic diseases include allergic rhinitis, contact dermatitis, eczema, and food allergies. Allergic skin reactions may also occur in some individuals as a result of exposure to latex. Symptoms of eczema, which is also referred to as atopic dermatitis, include skin lesions and/or dry skin typically on the face and extensor sides of arms and/or legs which may show extensive oozing and crusting, itching, erythema, and papules. Symptoms of contact dermatitis, such as allergic contact dermatitis, include an inflammation of the skin characterized by a rash including redness, itching, blistering, and in some instances scales on the skin. Symptoms of allergic rhinitis and hayfever include rhinorrhea, nasal itching, nasal obstruction, sneezing, wheezing, congestion, and itching of the throat and/or eyes. Hayfever is typically a seasonal condition resulting from allergy to grasses and pollens, and is oftentimes characterized by sneezing, or in more severe cases, inflammation and the development of polyps in the nasal passages. Symptoms of food allergies may manifest in the skin and/or in the respiratory tract. For instance, symptoms of food allergies may include hives (acute urticaria), swelling of the eyes, lips, and/or tongue (i.e., angioedema), eczema, allergic rhinoconjunctivitis, and anaphylaxis. Symptoms associated with latex allergies include allergic contact dermatitis, contact urticaria, rhinosinusitis, conjunctivitis, and asthma, among others.

In one aspect of the disclosure, administration of the compositions of the present disclosure to an individual in need thereof results in a decrease in respiratory distress when the individual is exposed to an allergen, as compared to individuals not administered the composition. The degree of respiratory distress may be determined by measuring the arterial oxygen pressure (pO₂) of the individual, as discussed in the examples, and/or by measuring the lung function of the individual, using any suitable technique known in the art. Thus, in one aspect, administration of the compositions of the present disclosure to an individual in need thereof results in a less severe drop in pO₂ levels when the individual is exposed to an allergen, as compared to individuals exposed to the allergen but not administered the composition. In another aspect, administration of the compositions of the present disclosure to an individual in need thereof decreases the changes in lung function (pleural pressure is a measure of lung function) of the individual when the individual is exposed to an allergen, as compared to individuals exposed to an allergen but not administered the composition

### METHODS OF MANUFACTURE

The nutritional base formulas of the present disclosure (into which the prebiotic and probiotic are introduced) may be prepared by any known or otherwise effective technique suitable for making and formulating a nutritional formula or similar other formula, variations of which may depend upon variables such as the selected product form, ingredient combination, packaging and container selection, and so forth, for the desired nutritional formula. Such techniques and variations for any given formula are easily determined and applied by one of ordinary skill in the nutritional formulation or manufacturing arts.

The nutritional base formulas of the present disclosure, including the exemplified formulas described hereinafter, can therefore be prepared by any of a variety of known or otherwise effective formulation or manufacturing methods. These methods most typically involve the initial formation of an aqueous slurry containing carbohydrates, proteins, lipids, stabilizers or other formulation aids, vitamins, minerals, or combinations thereof. The slurry is emulsified, pasteurized, homogenized, and cooled. Various other solutions, mixtures, or other materials may be added to the resulting emulsion before, during, or after further processing. This emulsion can then be further diluted, heat-treated, and packaged to form a ready-to-feed or concentrated liquid, or it can be heat-treated and subsequently processed and packaged as a reconstitutable powder, e.g., spray dried, dry mixed, agglomerated.

Other suitable methods for making nutritional formulas are described, for example, in U.S. Pat. No. 6,365,218 (Borschel, et al.), U.S Patent 6,589,576 (Borschel, et al.), U.S. Pat. No. 6,306,908 (Carlson, et al.), U.S. Patent Application 20030118703 A1 (Nguyen, et al.).

Once the nutritional base formula has been produced, the probiotic as described above may be dryblended into the nutritional base formula to produce the nutritional formulas of the present disclosure. The probiotic is introduced into the nutritional base formula and thoroughly mixed into the nutritional base formula using suitable conventional mixing equipment to produce a substantially homogenous nutritional formula.

### EXAMPLES

The following examples further describe and demonstrate specific embodiments within the scope of the present disclosure. All exemplified amounts are weight percentages based upon the total weight of the composition, unless otherwise specified.

Each of the exemplified formulas is fed to humans to provide sole, primary, or supplemental nutrition. Each formula contains a synbiotic composition of the present disclosure, wherein each composition may be used for the prevention and/or treatment of allergic disease.

### Examples 1-13

The following examples illustrate nutritional infant formulas of the present disclosure, including methods of making and using the infant formulas. Formula ingredients for each batch are listed in Tables 2 and 3.

**Table 2: Nutritional Infant Formulas Comprising FOS or GOS**

| **Ingredients** | **Example 1** | **Example 2** | **Example 3** | **Example 4** | **Example 5** | **Example 6** |
|---|---|---|---|---|---|---|
| | Quantity (lbs) per 1000 lbs | Quantity (lbs) per 1000 lbs | Quantity (lbs) per 1000 lbs | Quantity (lbs) per 1000 lbs | Quantity (lbs) per 1000 lbs | Quantity (lbs) per 1000 lbs |
| Non Fat Dry Milk | 204.43 | 204.43 | 204.43 | 204.43 | 204.43 | 204.43 |
| Lactose | 381.80 | 366.42 | 351.03 | 381.80 | 366.42 | 351.03 |
| High Oleic Safflower Oil | 116.62 | 116.62 | 116.62 | 116.62 | 116.62 | 116.b2 |
| Soy Oil | 88.59 | 88.59 | 88.59 | 88.59 | 88.59 | 88.59 |
| Coconut Oil | 81.60 | 81.60 | 81.60 | 81.60 | 81.60 | 81.60 |
| Galactooligosaccharides (GOS) | --- | --- | --- | 15.38 | 46.15 | 76.92 |
| Fructooligosaccharides (FOS) | 15.38 | 46.15 | 76.92 | --- | --- | --- |
| Whey Protein Concentrate | 50.31 | 50.31 | 50.31 | 50.31 | 50.31 | 50.31 |
| Potassium Citrate (1) | 5.76 | 5.76 | 5.76 | 5.76 | 5.76 | 5.76 |
| Calcium Carbonate | 4.06 | 4.06 | 4.06 | 4.06 | 4.06 | 4.06 |
| Arachidonic Acid | 2.95 | 2.95 | 2.95 | 2.95 | 2.95 | 2.95 |
| Potassium Chloride | 1.26 | 1.26 | 1.26 | 1.26 | 1.26 | 1.26 |
| Lecithin Ultralec | 1.15 | 1.15 | 1.15 | 1.15 | 1.15 | 1.15 |
| Docosahexaenoic Acid | 1.11 | 1.11 | 1.11 | 1.11 | 1.11 | 1.11 |
| Magnesium Chloride | 1.04 | 1.04 | 1.04 | 1.04 | 1.04 | 1.04 |
| Sodium Chloride | 0.59 | 0.59 | 0.59 | 0.59 | 0.59 | 0.59 |
| Choline Chloride | 0.43 | 0.43 | 0.43 | 0.43 | 0.43 | 0.43 |
| Vitamin ADEK | 0.39 | 0.39 | 0.39 | 0.39 | 0.39 | 0.39 |
| Ascorbyl Palmitate | 0.37 | 037 | 0.37 | 0.37 | 0.37 | 0.37 |
| Mixed Carotenoid Premix | 0.35 | 0.35 | 0.35 | 0.35 | 0.35 | 0.35 |
| Mixed Tocopherols | 0.16 | 0.16 | 0.16 | 0.16 | 0.16 | 0.16 |
| Ascorbic Acid | 1.28 | 1.28 | 1.28 | 1.28 | 1.28 | 1.28 |
| Potassium Citrate (3) | 3.43 | 3.43 | 3.43 | 3.43 | 3.43 | 3.43 |
| Riboflavin | 0.003 | 0.003 | 0.003 | 0.003 | 0.003 | 0.003 |
| L-Carnitine | 0.026 | 0.026 | 0.026 | 0.026 | 0.026 | 0.026 |
| Vitamin/Mineral Premix | 1.12 | 1.12 | 1.12 | 1.12 | 1.12 | 1.12 |
| Ferrous Sulfate | 0.45 | 0.45 | 0.45 | 0.45 | 0.45 | 0.45 |
| Potassium Citrate (2) | 0.026 | 0.026 | 0.026 | 0.026 | 0.026 | 0.026 |
| Nucleotide/Choline Premix | 2.35 | 2.35 | 2.35 | 2.35 | 2.35 | 2.35 |
| *L. rhamnosus* HN001 (DR20)* | 5x10¹² CFUs | 5x10¹³ CFUs | 5x10¹¹ CFUs | 5X10¹² CFUs | 5x10¹³ CFUs | 5x10¹⁴ CFUs |
| *Units are in CFUs per 1000 lbs | | | | | | |

**Table 3: Nutritional Infant Formulas Comprising HMOs, GOS, and/or FOS**

| **Ingredients** | **Example 7** | **Example 8** | **Example 9** | **Example 10** | **Example 11** | **Example 12** | **Example 13** |
|---|---|---|---|---|---|---|---|
| | Quantity (lbs) per 1000 lbs | Quantity (lbs) per 1000 lbs | Quantity (lbs) per 1000 lbs | Quantity (lbs) per 1000 lbs | Quantity (lbs) per 1000 lbs | Quantity (lbs) per 1000 lbs | Quantity (lbs) per 1000 lbs |
| Non Fat Dry Milk | 204.43 | 204.43 | 204.43 | 204.43 | 204.43 | 204.43 | 204.43 |
| Lactose | 389.30 | 387.57 | 370.26 | 389.49 | 366.42 | 366.42 | 389.49 |
| High Oleic Safflower Oil | 116.62 | 116.62 | 116.62 | 116.62 | 116.62 | 116.62 | 116.62 |
| Soy Oil | 88.59 | 88.59 | 88.59 | 88.59 | 88.59 | 88.59 | 88.59 |
| Coconut Oil | 81.60 | 81.60 | 81.60 | 81.60 | 81.60 | 81.60 | 81.60 |
| Galactooligosaccharides (GOS) | --- | --- | --- | | 46.15 | --- | |
| Fructooligosaccharides (FOS) | --- | --- | --- | | --- | 46.15 | |
| Human Milk Oligosaccharides (HMO) | 0.38 | 3.84 | 38.46 | --- | 3.84 | 3.84 | |
| Whey Protein Concentrate | 50.31 | 50.31 | 50.31 | 50.31 | 50.31 | 50.31 | 50.31 |
| Potassium Citrate (1) | 5.76 | 5.76 | 5.76 | 5.76 | 5.76 | 5.76 | 5.76 |
| Calcium Carbonate | 4.06 | 4.06 | 4.06 | 4.06 | 4.06 | 4.06 | 4.06 |
| Arachidonic Acid | 2.95 | 2.95 | 2.95 | 2.95 | 2.95 | 2.95 | 2.95 |
| Potassium Chloride | 1.26 | 1.26 | 1.26 | 1.26 | 1.26 | 1.26 | 1.26 |
| Lecithin Ultralec | 1-15 | 1.15 | 1.15 | 1.15 | 1.15 | 1.15 | 1.15 |
| Docosahexaenoic Acid | 1.11 | 1.11 | 1.11 | 1.11 | 1.11 | 1.11 | 1.11 |
| Magnesium Chloride | 1.04 | 1.04 | 1.04 | 1.04 | 1.04 | 1.04 | 1.04 |
| Sodium Chloride | 0.59 | 0.59 | 0.59 | 0.59 | 0.59 | 0.59 | 0.59 |
| Choline Chloride | 0.43 | 0.43 | 0.43 | 0.43 | 0.43 | 0.43 | 0.43 |
| Vitamin ADEK | 0.39 | 0.39 | 0.39 | 0.39 | 0.39 | 0.39 | 0.39 |
| Ascorbyl Palmitate | 0.37 | 0.37 | 0.37 | 0.37 | 0.37 | 0.37 | 0.37 |
| Mixed Carotenoid Premix | 0.35 | 0.35 | 0.35 | 0.35 | 0.35 | 0.35 | 0.35 |
| Mixed Tocopherols | 0.16 | 0.16 | 0.16 | 0.16 | 0.16 | 0.16 | 0.16 |
| Ascorbic Acid | 1.28 | 1.28 | 1.28 | 1.28 | 1.28 | 1.28 | 1.28 |
| Potassium Citrate (3) | 3.43 | 3.43 | 3.43 | 3.43 | 3.43 | 3.43 | 3.43 |
| Riboflavin | 0.003 | 0.003 | 0.003 | 0.003 | 0.003 | 0.003 | 0.003 |
| L-Carnitine | 0.026 | 0.026 | 0.026 | 0.026 | 0.026 | 0.026 | 0.026 |
| Vitamin/Mineral Premix | 1.12 | 1.12 | 1.12 | 1.12 | 1.12 | 1.12 | 1.12 |
| Ferrous Sulfate | 0.45 | 0.45 | 0.45 | 0.45 | 0.45 | 0.45 | 0.45 |
| Potassium Citrate (2) | 0.026 | 0.026 | 0.026 | 0.026 | 0.026 | 0.026 | 0.026 |
| Nucleotide/Choli ne Premix | 2.35 | 2.35 | 2.35 | 2.35 | 2.35 | 2.35 | 2.35 |
| *L. rhamnosus* HN001 (DR20)* | 5x10¹² CFUs | 5x10¹³ CFUs | 5x10¹⁴ CFUs | | 5x10¹³ CFUs | 5x10¹³ CFUs | |
| Units are in CFUs per 1000 lbs | | | | | | | |

The exemplified formulas are prepared as a powdered nutritional infant formula by making at least two separate slurries that are later blended together, heat treated, standardized, heat treated a second time, spray dried, agglomerated, dry blended, and packaged, or may be prepared as a liquid ready-to-feed infant nutritional formula by making at least two separate slurries that are later blended together, heat treated, standardized, diluted with an appropriate amount of water, packaged, and sterilized. Initially, a carbohydrate-mineral slurry is prepared by dissolving the carbohydrates and prebiotics in water at 60-71°C, followed by the addition of magnesium chloride, choline chloride, and sodium chloride, potassium chloride, and calcium carbonate. The resulting slurry is held under moderate agitation at 49-60°C until it is later blended with the other prepared slurries.

An oil slurry is prepared by combining high oleic safflower oil, soy oil, coconut oil, arachidonic acid, and docosahexaenoic acid at 49-60°C, followed by the addition of ascorbyl palmitate, mixed tocopherols, mixed carotenoid premix, vitamin ADEK premix, and lecithin. The resulting oil slurry is held under moderate agitation at 38-49°C until it is later blended with the other prepared slurries.

A protein slurry is prepared by dissolving non-fat dry milk and whey protein concentrate in water at approximately 5-30°C. The resulting protein slurry is held under low agitation at 2-7°C until it is later blended with the other prepared slurries.

Water, the carbohydrate-mineral slurry, and the protein slurry are combined under adequate agitation. The oil slurry is then added. The pH of the resulting blend is adjusted with potassium hydroxide. This blend is held under moderate agitation at 49-60°C.

The resulting blend is heated to 71-77°C, emulsified to a maximum of 300 psig, and then homogenized at 2400-2600/400-600 psig. The blend is then heated to 144-146°C for about 5 seconds. The heated blend is then cooled to a temperature of about 4°C. Samples are taken for microbiological and analytical testing. The mixture is held under agitation.

A vitamin/mineral premix solution and an ascorbic acid solution are prepared separately and added to the processed blended slurry. The vitamin/mineral premix solution is prepared by adding the following ingredients to water with agitation: potassium citrate, ferrous sulfate, vitamin/mineral premix, L-carnitine, riboflavin, and the nucleotide-choline premix. The ascorbic acid solution is prepared by adding potassium hydroxide and ascorbic acid to a sufficient amount of water to dissolve the ingredients. The ascorbic acid solution pH is then adjusted to 5-9 with potassium hydroxide.

To prepare a powdered nutritional infant formula, the blend pH may be adjusted with potassium hydroxide to achieve optimal product stability. The blend then receives a second heat treatment. The blend is originally heated to 71-77°C, and then further heated to 144-146°C for about 5 seconds. The heated blend is then passed through a flash cooler to reduce the temperature to 71-82°C. Following heat treatment, the blend is evaporated.

The evaporated blend is passed through a spray drier. The finished powder then undergoes agglomeration with water as the binder solution. The probiotic is dryblended into the product. The completed product is then packaged into suitable containers.

To prepare a ready-to-feed nutritional infant formula, based on the analytical results of the quality control tests, an appropriate amount of water is added to the batch with agitation to achieve the desired total solids. The product pH may be adjusted to achieve optimal product stability. The completed product is then placed in suitable containers and subjected to terminal sterilization.

The resulting formula is then used to provide a supplemental, primary, or sole source of nutrition to infants or other appropriate individuals.

### EXPERIMENT 1

A study was conducted to evaluate the ability of a synbiotic composition comprising the probiotic *Lactobacillus rhamnosus* HN001 (DR20^{™}) and a fructooligosaccharide-based prebiotic to treat and/or prevent allergic diseases, as compared to compositions comprising only *Lactobacillus rhamnosus* HN001, compositions comprising only a fructooligosaccharide-based prebiotic, and control compositions comprising neither *Lactobacillus rhamnosus* HN001 nor a fructooligosaccharide-based prebiotic.

The study evaluated the effects of the compositions in a pig allergic lung disease model. A pig model is a more relevant animal model than mouse models, due to the immune similarities between human and pig. Specifically, type 1 cytokine responses of piglets develop over time like that of human infants (Diaz, et al., "Use of ELISPOT and ELISA to evaluate IFN-γ, IL-10 and IL-4 responses in conventional pigs," Vet. Immunol. Immunopathol., 2005, Vol. 106, p. 107-112; Buck, et al., "Longitudinal study of intracellular T cell cytokine production in infants compared to adults," Clin. Exp. Immunol., 2002, Vol. 128, p. 490-497). Furthermore, the cytokine response of pigs to infectious pathogens is also comparable to humans. For instance, it has been shown that *Toxoplasma gondii* elicits a type 1 response and *Ascaris suum* elicits a type 2 response in pigs (Dawson, et al., "Localized multigene expression patterns support an evolving TH1/TH2-like paradigm in response to infections with Toxoplasma gondii and Ascaris suum," Infect. Immun., 2005, Vol. 73, p. 1116-1128). Similarities between humans and pigs have also been noted in a subset of antigen presenting dendritic cells known as plasmacytoid dendritic cells, whereas mice and humans differ (Hochrein, et al., "Of men, mice and pigs: looking at their plasmacytoid dendritic cells," Immunology, 2004, Vol. 112, p. 26-27). Since these immune functions may play a role in the pathogenesis of allergic lung disease, a pig model is a relevant animal model.

Pigs were evaluated over a 70 day period. Specifically, pigs from the University of Illinois Research Farm (Urbana, Illinois) were sow reared to 21 days of life, at which time they were weaned. When weaned, pigs were fed either normal chow (University of Illinois Feed Mill), normal chow supplemented with *Lactobacillus rhamnosus* HN001 (10¹⁰ CFU daily), normal chow supplemented with a fructooligosaccharide-based prebiotic (4% wt/wt, Synergy 1, Orafti), or normal chow supplemented with a synbiotic composition comprising *Lactobacillus rhamnosus* HN001 (10¹⁰ CFU daily) and a fructooligosaccharide-based prebiotic (4% wt/wt, Synergy 1, Orafti), for the remainder of the 70 day study. During this time period, the pigs were exposed to an allergen, *Ascaris suum* antigen (ASA) subcutaneously (3x at study day 21,35, and 49) and 1 mg of ASA intranasally (2x at study day 49 and 63). The pigs were challenged intradermally on day 45 and day 70 with a series of concentrations of ASA ranging from 0.35 µg/mL to 400 µg/mL to determine the effects of oral supplementation with the probiotic on immediate hypersensitivity skin reactions (IgE mediated reactions). On day 70, pigs were also intranasally challenged with ASA to determine the effects of oral supplementation with the probiotic, prebiotic, and synbiotic on allergic lung disease.

Table 4 below summarizes the number of pigs in each group experiencing respiratory distress of any magnitude and Table 5 summarizes the severity of the respiratory distress of the pigs in each group.

**Table 4: Incidence of respiratory distress**

| | **Control (n=5)** | **Probiotic (n=6)** | **Prebiotic (n=6)** | **Synbiotic (n=6)** |
|---|---|---|---|---|
| Number of pigs experiencing respiratory distress | 4 | 2 | 4 | 1 |
| Number of pigs experiencing no respiratory distress | 1 | 4 | 2 | 5 |

**Table 5: Severity of respiratory distress**

| | **Control (n=5)** | **Probiotic (n=6)** | **Prebiotic (n=6)** | **Synbiotic (n=6)** |
|---|---|---|---|---|
| Number of pigs experiencing severe/extreme respiratory distress | 1 | 1 | 1 | 1 |
| Number of pigs experiencing moderate/mild respiratory distress | 3 | 1 | 3 | 0 |
| Number of pigs experiencing no respiratory distress | 1 | 4 | 2 | 5 |

The severity of pig respiratory distress was stratified based on partial oxygen pressures (pO₂, see below). Specifically, pO₂ levels were tested at 2 minutes, 5 minutes, 10 minutes, 20 minutes, and 30 minutes post-respiratory challenge. If the pO₂ did not drop below 70 at any point following challenge, the severity was classified as no respiratory distress. No pO₂ drop below 60 at any point followed by a quick recovery was classified as mild respiratory distress, a pO₂ drop below 60 and sustained anoxia was classified as moderate respiratory distress, a pO₂ drop below 60 and sustained anoxia requiring recovery therapy (Amubu bag™ bag valve mask) was classified as severe respiratory distress, and a reaction that was not responsive to recovery therapy was determined to be extreme respiratory distress.

Figure 1 shows the average change in pO₂ levels (mean + SEM) from baseline following respiratory challenge. pO₂ levels are an indication of how well the lungs are functioning to oxygenate the blood. As can be seen from Figure 1, there was a larger immediate drop in the pO₂ levels in the control group following challenge, as compared to the probiotic, prebiotic, and synbiotic groups. Starting at 5 minutes post-respiratory challenge, the synbiotic group exhibited the lowest drop in pO₂ levels, as compared to the other groups tested.

The pleural pressure of the pigs was also tested at 2 minutes, 5 minutes, 10 minutes, 20 minutes, and 30 minutes following respiratory challenge. An increase in pleural pressure indicates greater force is needed to expand and contract the lungs, and therefore is indicative of impaired lung function. Figure 2 depicts the change in pleural pressure (mean + SEM) from baseline among the various groups following respiratory challenge. As can be seen from Figure 2, there was a smaller increase in pleural pressure for the probiotic and synbiotic groups as compared to the control group.

Bronchoalveolar lavage fluid taken from the pigs in each group 24 hours post-respiratory challenge was tested for cytokine levels. The results are shown in Figure 3. As can be seen from Figure 3, inflammatory cytokines (IL-1β, IL-8, and TNF-α) were higher in the control, probiotic, and prebiotic groups as compared to the synbiotic group. IL-2 is also higher in the control group and prebiotic compared to the synbiotic group. It has previously been demonstrated that IL-2 producing T cells are found in the bronchial biopsies of allergen challenged asthmatics (Bousquet, J, Jeffery PK, Busse WW, Johnson M, Vignola AM, Asthma From Bronchoconstriction To Airways Inflammation and Remodeling. AM J Respir Crit Care Med 2000; 161: 1720-1745).

### EXPERIMENT 2

A similar experiment to Experiment 1 was conducted. In Experiment 2, pigs from the University of Illinois Research Farm (Urbana, Illinois) were sow reared to 21 days of life, at which time they were weaned. After weaning, pigs were fed either normal chow (University of Illinois Feed Mill), normal chow supplemented with *Lactobacillus* rhamnosus HN001 (10¹⁰ CFU daily), normal chow supplemented with a galactooligosaccharide-based prebiotic (4% wt/wt, Vivinal GOS, Domo), or normal chow supplemented with a symbiotic composition comprising *Lactobacillus rhamnosus* HN001 (10¹⁰ CFU daily) and a galactooligosaccharide-based prebiotic (4% wt/wt, Vivinal GOS, Friesland). The effects of the control, probiotic, prebiotic and synbiotic compositions on allergic skin flare reactions are depicted in Figure 4. Specifically, Figure 4 depicts the allergic skin flare reactions 20 minutes after challenge with the differing doses of challenge antigen on day 45 for pigs administered the control composition as compared to pigs administered the probiotic, prebiotic, and the symbiotic compositions. As can be seen from these results, pigs administered the control chow developed larger allergic skin flare reactions when challenged at day 45 with 12 µg/mL and higher doses of ASA. In contrast, pigs administered the probiotic composition and prebiotic composition had smaller allergic skin flare reactions compared to the control pigs while pigs administered the symbiotic composition had the smallest skin flare reactions 20 minutes after the day 45 challenge.

### CONCLUSION

The data set forth herein shows that a synbiotic composition comprising the probiotic *Lactobacillus rhamnosus* HN001 and a fructooligosaccharide-based prebiotic reduced incidences and severity of respiratory distress when administered to pigs, as compared to control pigs administered normal chow and pigs administered the probiotic alone or the prebiotic alone. Pigs administered the synbiotic composition also exhibited the lowest drop in pO₂ levels starting at 5 minutes post-respiratory challenge, exhibited a smaller increase in pleural pressure starting at 10 minutes post-respiratory challenge, and had lower levels of the inflammatory cytokines IL-1β, IL-8, IL-2, and TNF-α in their bronchoalveolar lavage fluid at 24 hours post-respiratory challenge, as compared to the other groups tested. Moreover, the data shows that the pigs administered the symbiotic containing *Lactobacillus rhamnosus* HN001 and a galactooligosaccharide composition had smaller skin flare reactions 20 minutes after the day 45 challenge for all doses of ASA tested as compared to pigs administered the control chow only, indicating that the probiotic *Lactobacillus rhamnosus* HN001 in combination with a prebiotic is effective in preventing or reducing the incidence of allergic skin flares.

## Claims

1. A composition for use in treating or preventing allergic lung disease, comprising:
a probiotic comprising *Lactobacillus rhamnosus* HN001, and
a prebiotic comprising fructooligosaccharide.

2. The composition for use according to claim 1 comprising the prebiotic in an amount of from 1.5 grams prebiotic per 100 grams composition to 9.2 grams prebiotic per 100 grams composition.

3. The composition for use according to claim 1 comprising probiotic in an amount of from 10⁴ cfu of probiotic per gram of composition to 10¹⁰ cfu of probiotic per gram of composition.

4. The composition for use according to claim 1 further comprising at least one of vitamins, minerals, carbohydrate, fat, and protein.

5. The composition for use according to claim 4 comprising, as a percentage of total calories, 10% to 70% carbohydrate, 5% to 50% lipid component, and 10% to 80% protein.

6. The composition for use according to claim 1 wherein the composition is a powdered infant formula.

7. The composition for use according to claim 1 wherein the composition is a ready-to-feed liquid infant formula.

8. An infant formula comprising a probiotic comprising *Lactobacillus rhamnosus* HN001, and a prebiotic selected from the group consisting of fructooligosaccharides, galactooligosaccharides, and combinations thereof, for use in a method of treating or preventing allergic lung disease in an individual in need of such treatment or prevention, the method comprising administering to the individual an effective amount of said infant formula.

9. The infant formula for use according to claim 8 wherein the allergic lung disease is selected from the group consisting of asthma, hayfever, and hypersensitive pneumonitis.

10. The infant formula for use according to claim 9 wherein the allergic lung disease is asthma.

11. The infant formula for use according to claim 8 wherein the infant formula is administered prior to exposing the individual to an allergen.

## Patentansprüche

1. Zusammensetzung zur Verwendung bei der Behandlung oder Vorbeugung von allergischer Lungenkrankheit, die Folgendes aufweist:
ein Probiotikum, das *Lactobacillus rhamnosus* HN001 aufweist, und
ein Präbiotikum, das Fructooligosaccharid aufweist.

2. Zusammensetzung zur Verwendung nach Anspruch 1, die das Präbiotikum in einer Menge von von 1,5 Gramm Präbiotikum pro 100 Gramm der Zusammensetzung bis 9,2 Gramm Präbiotikum pro 100 Gramm der Zusammensetzung aufweist.

3. Zusammensetzung zur Verwendung nach Anspruch 1, die das Probiotikum in einer Menge von von 10⁴ KbE des Probiotikums pro Gramm der Zusammensetzung bis 10¹⁰ KbE des Probiotikums pro Gramm der Zusammensetzung aufweist.

4. Zusammensetzung zur Verwendung nach Anspruch 1, die ferner wenigstens eines der Folgenden aufweist: Vitamine, Mineralien, Kohlenhydrat, Fett und Protein.

5. Zusammensetzung zur Verwendung nach Anspruch 4, die, als Prozentsatz der gesamten Kalorien, 10% bis 70% Kohlenhydrat, 5% bis 50% eines Lipidbestandteils und 10% bis 80% Protein aufweist.

6. Zusammensetzung zur Verwendung nach Anspruch 1, wobei die Zusammensetzung eine pulverisierte Säuglingsanfangsnahrung ist.

7. Zusammensetzung zur Verwendung nach Anspruch 1, wobei die Zusammensetzung eine fütterungsfertige flüssige Säuglingsanfangsnahrung ist.

8. Säuglingsanfangsnahrung mit einem Probiotikum, das *Lactobacillus rhamnosus* HN001 aufweist, und mit einem Präbiotikum, das aus der Gruppe bestehend aus Fructooligosacchariden, Galactooligosacchariden und Kombinationen davon ausgewählt ist, zur Verwendung in einem Verfahren zur Behandlung oder Vorbeugung von allergischer Lungenkrankheit bei einer Person, die eine derartige Behandlung oder Vorbeugung benötigt, wobei das Verfahren ein Verabreichen an die Person einer effektiven Menge der Säuglingsanfangsnahrung aufweist.

9. Säuglingsanfangsnahrung zur Verwendung nach Anspruch 8, wobei die allergische Lungenkrankheit aus der Gruppe bestehend aus Asthma, Heuschnupfen und hypersensitiver Pneumonitis ausgewählt ist.

10. Säuglingsanfangsnahrung zur Verwendung nach Anspruch 9, wobei die allergische Lungenkrankheit Asthma ist.

11. Säuglingsanfangsnahrung zur Verwendung nach Anspruch 8, wobei die Säuglingsanfangsnahrung verabreicht wird, bevor die Person einem Allergen ausgesetzt wird.

## Revendications

1. Composition destinée à être utilisée dans le traitement ou la prévention d'une maladie pulmonaire allergique, comprenant :
un probiotique comprenant *Lactobacillus rhamnosus* HN001, et
un prébiotique comprenant un fructo-oligosaccharide.

2. Composition destinée à être utilisée suivant la revendication 1, comprenant le prébiotique en une quantité de 1,5 gramme de prébiotique pour 100 grammes de composition à 9,2 grammes de prébiotique pour 100 grammes de composition.

3. Composition destinée à être utilisée suivant la revendication 1, comprenant le probiotique en une quantité de 10⁴ cfu de probiotique par gramme de composition à 10¹⁰ cfu de probiotique par gramme de composition.

4. Composition destinée à être utilisée suivant la revendication 1, comprenant en outre au moins une de vitamines, de substances minérales, d'une substance glucidique, d'une matière grasse et d'une protéine.

5. Composition destinée à être utilisée suivant la revendication 4, comprenant, en pourcentage des calories totales, 10% à 70% de glucides, 5% à 50% de constituant lipidique, et 10% à 80% de protéines.

6. Composition destinée à être utilisée suivant la revendication 1, la composition étant une formulation en poudre pour nourrissons.

7. Composition destinée à être utilisée suivant la revendication 1, la composition étant une formulation liquide pour nourrissons, prête à la consommation.

8. Formulation pour nourrissons comprenant un probiotique comprenant *Lactobacillus rhamnosus* HN001, et un prébiotique choisi dans le groupe consistant en des fructooligosaccharides, des galacto-oligosaccharides et leurs associations, destinée à être utilisée dans un procédé de traitement ou de prévention d'une maladie pulmonaire allergique chez un individu ayant besoin d'un tel traitement ou d'une telle prévention, le procédé comprenant l'administration à l'individu d'une quantité efficace de ladite formulation pour nourrissons.

9. Formulation pour nourrissons destinée à être utilisée suivant la revendication 8, la maladie pulmonaire allergique étant choisie dans le groupe consistant en l'asthme, le rhume des foins et la pneumonie d'hypersensibilité.

10. Formulation pour nourrissons destinée à être utilisée suivant la revendication 9, la maladie pulmonaire allergique étant l'asthme.

11. Formulation pour nourrissons destinée à être utilisée suivant la revendication 8, la formulation pour nourrissons étant administrée avant exposition de l'individu à un allergène.
